# EUROPÄISCHE PATENTSCHRIFT

(11) **EP 1 177 438 B1**
(45) Veröffentlichungstag und Bekanntmachung des Hinweises auf die Patenterteilung: **05.11.2003**
(21) Anmeldenummer: 00941914.4
(22) Anmeldetag: 11.05.2000
(51) Int. Cl.: G01N 33/483, G01N 33/46, G01N 33/38, G01N 29/10

(54) **VORRICHTUNG ZUR MATERIALUNTERSUCHUNG**
DEVICE FOR INVESTIGATING MATERIALS
DISPOSITIF POUR ESSAIS DE MATERIAUX

(30) Priorität: 11.05.1999 DE 19921568
(43) Veröffentlichungstag der Anmeldung: 06.02.2002
(73) Patentinhaber: Rinn, Frank, 69126 Heidelberg (DE)
(72) Erfinder: Rinn, Frank, 69126 Heidelberg (DE)
(74) Vertreter: Maisch, Thomas, Dr. rer. nat.
(86) Internationale Anmeldenummer: DE0001467
(87) Internationale Veröffentlichungsnummer: WO00068682

(56) Entgegenhaltungen:
- EP-A- 0 448 896
- DD-A- 206 908
- FR-A- 2 662 502
- US-A- 3 292 143
- US-A- 3 877 294
- US-A- 4 926 691
- US-A- 4 979 124
- US-A- 5 621 172

## Beschreibung

Die Erfindung betrifft eine Vorrichtung zur Materialuntersuchung, insbesondere von Bäumen, sonstigen Hölzern und Beton, mit einem Impulsgeber zur Erzeugung eines in das Material einleitbaren Impulses, mindestens einem dem Material zuordenbaren Sensor zur Detektion des Impulses und einer Auswerteelektronik zur Diskriminierung des Impulses von Störimpulsen gemäß den Ansprüchen 1-13.

Vorrichtungen zur Materialuntersuchung der eingangs genannten Art sind aus der Praxis bekannt und existieren in den unterschiedlichsten Ausführungsformen. Dabei handelt es sich beispielsweise um Vorrichtungen, bei denen die Impulslaufzeit von Stoßwellen gemessen wird. Aus dieser Impulslaufzeit werden Rückschlüsse auf die Qualität des zu untersuchenden Materials gezogen. Bei Holz, beispielsweise Telegraphenmasten, korreliert die Laufzeit der Stoßwelle in Wuchs- bzw. Faserlängsrichtung mit dem Biege-E-Modul des Holzes, was eine Abschätzung der Belastbarkeit und die davon abhängige Einordnung in Güteklassen ermöglicht. Hierdurch wird bei neuen Masten der Kaufpreis beeinflußt. Vorrichtungen solcher Art sind in US 5 621 172 und US 4 926 691 beschrieben.

Die Stoßwelle bzw. der Impuls wird hierbei meist mit einem als Impulsgeber dienenden Hammer entweder direkt oder über eine Schraube oder einen Schlagstift in das Material eingeleitet. Bei einer Längsdurchschallung eines Masten erfolgt die Impulseinleitung meist stimseitig. Ein am anderen Ende des Materials bzw. Masten angeordneter Sensor detektiert den in das Material eingeleiteten Impuls. Ein diesem Impuls entsprechender Stromimpuls wird dann vom Sensor zu einer zentralen Auswerteelektronik geleitet. Dort wird der Stromimpuls analysiert, wobei Störimpulse diskriminiert werden.

Genauer gesagt sendet ein Beschleunigungssensor im impulsgebenden Hammer im Moment des Schlageintrags den resultierenden Stromimpuls über eine Leitung an die zentrale elektronische Einheit bzw. Auswerteelektronik, die den Impuls analysiert und je nach Ergebnis - erfolgreicher Diskriminierung von beispielsweise störenden Erschütterungen - eine Uhr startet. Sobald der Sensor am anderen Ende des Materials bzw. am anderen Ende der Meßstrecke das Ankommen der Stoßwelle registriert, sendet auch er einen entsprechenden Stromimpuls zur zentralen elektronischen Einheit, die die Uhr stoppt, falls der Impuls den Anforderungen bezüglich Intensität und Länge entspricht. Sowohl der Impuls vom Hammer als auch der Impuls vom Sensor müssen jeweils elektronisch diskriminiert, d. h. von anderen Erschütterungen unterschieden werden. Dies erfolgt jeweils in der zentralen Auswerteelektronik. Damit echte Impulse und Fehlimpulse unterschieden werden, können in der Regel "Gain" und "Offset" an der zentralen Auswerteelektronik vom Anwender eingestellt werden. Aus der Laufzeit des Impulses und der Entfernung zwischen Schlageintragung und Detektor kann die Impuls- bzw. Stoßwellengeschwindigkeit bestimmt werden. Sie ermöglicht, nicht nur bei Holz, sondern auch bei Beton und anderen Materialien, Aussagen über den inneren Zustand und die Qualität des zu untersuchenden Materials bzw. Prüflings.

Bei der bekannten Vorrichtung werden die elektronischen Signale der Beschleunigungssensoren der Impulsgeber und der Detektoren über Kabel zu einer zentralen Erkennungs- und Auswerteelektronik gesendet. Dort ist auch eine genaue elektronische Uhr angeordnet. Die Diskriminierung und Auswertung der in elektronische Stromimpulse gewandelten, ehemals eingeleiteten und anschließend detektierten Impulse erfolgt also an einer zentralen Stelle mittels entsprechender elektronischer Schaltungen. Die Impulsform ist dabei entscheidend für die Unterscheidung zwischen echten Impulsen und Störimpulsen. Die Impulsform darf also auf dem Weg vom Sensor durch das Kabel zur Auswerteelektronik nicht durch beispielsweise elektromagnetische Störungen oder technische Kabeleigenschaften verändert oder verfälscht werden. Um dies zu erreichen, müssen die Übertragungskabel abgeschirmt sein und extrem hohe Qualität aufweisen, was zu hohen Preisen, begrenzter Länge von wenigen Metern und eingeschränkter Handhabung führt. Derartige Kabel mit entsprechender Abschirmung reagieren sehr empfindlich auf tiefe Temperaturen und andere äußere Effekte, so daß sie nur eingeschränkt anwendbar und sehr störanfällig sind. Beispielsweise dürfen derartige Kabel nicht in einer Schleife liegen, um Störungen zu vermeiden. Insbesondere bei sehr langen oder großen Prüflingen des zu untersuchenden Materials ist die bekannte Vorrichtung nicht einsetzbar, da keine ausreichend langen Kabel existieren, die eine störungsfreie Stromimpuls-Übertragung von dem Sensor oder den Sensoren zur zentralen Auswerteelektronik ermöglichen.

Folglich ist die Anwendung der bekannten Vorrichtung zur Materialuntersuchung insbesondere hinsichtlich großer Prüflinge an zu untersuchenden Materialien stark eingeschränkt. Eine universelle Verwendung der bekannten Vorrichtung ist daher nicht möglich.

Der vorliegenden Erfindung liegt daher die Aufgabe zugrunde, eine Vorrichtung zur Materialuntersuchung der eingangs genannten Art anzugeben, bei der eine universelle Anwendung, insbesondere auch bei großen Prüflingen des zu untersuchenden Materials, mit konstruktiv einfachen Mitteln ermöglicht ist.

Die voranstehende Aufgabe ist durch eine Vorrichtung zur Materialuntersuchung mit den Merkmalen des Patentanspruchs 1 gelöst. Danach ist eine Vorrichtung zur Materialuntersuchung der eingangs genannten Art derart ausgebildet, daß jedem Sensor eine Auswerteelektronik zugeordnet ist.

In erfindungsgemäßer Weise ist zunächst erkannt worden, daß die obige Aufgabe allein aufgrund geeigneter Anordnung der Auswerteelektronik auf überraschend einfache Weise gelöst ist. Hierzu ist in weiter erfindungsgemäßer Weise jedem Sensor eine separate Auswerteelektronik zugeordnet. Durch diese Zuordnung jeweils einer Auswerteelektronik zu jedem Sensor lassen sich insbesondere beim Einsatz mehrerer Sensoren lange Kabelstrecken zwischen den Sensoren und der Auswerteelektronik vermeiden. Hingegen sind derart lange Kabelstrecken beim Einsatz einer zentralen Auswerteelektronik für sämtliche eingesetzten Sensoren, die häufig mit großem Abstand zueinander angeordnet sind, für die meisten Sensoren nicht zu vermeiden.

Bei der erfindungsgemäßen Vorrichtung ist es nunmehr möglich, die Diskriminierung von Impulsen quasi unmittelbar am Sensor mit minimalen Kommunikationsstrecken durchzuführen. Da jedem Sensor eine Auswerteelektronik zugeordnet ist, spielen die Abstände zwischen den Sensoren keine Rolle mehr. Daher könnten die Sensoren auch mit großem Abstand zueinander angeordnet werden, wobei dennoch eine sichere Diskriminierung von Impulsen ermöglicht ist.

Folglich ist mit der erfindungsgemäßen Vorrichtung zur Materialuntersuchung eine Vorrichtung realisiert, bei der eine universelle Anwendung, insbesondere auch bei großen Prüflingen des zu untersuchenden Materials, mit konstruktiv einfachen Mitteln ermöglicht ist.

Im Hinblick auf die Art des Impulses bieten sich zwei Alternativen an. Dabei könnte der Impuls ein mechanischer und/oder elektrischer Impuls sein. Als mechanischer Impuls gilt beispielsweise eine Stoßwelle, die beispielsweise mittels eines Hammers ausgelöst wird. Es ist jedoch auch möglich elektrische Impulse in das zu untersuchende Material einzuleiten, wobei auch hier die Laufzeit des Stromimpulses und/oder seine Abschwächung gemessen werden kann. Dabei kann es sich um einen Gleichstrom- oder um einen Wechselstromimpuls handeln. Beim Wechselstromimpuls kann auch sein Frequenzverhalten während des Durchgangs durch das Material untersucht werden.

Im Hinblick auf eine besonders kurze und sichere Kommunikationsstrecke zwischen dem Sensor und der Auswerteelektronik könnte die Auswerteelektronik unmittelbar neben dem Sensor angeordnet oder in den Sensor integriert sein. Insbesondere die integrierte Anordnung der Auswerteelektronik in dem Sensor gewährleistet eine besonders kompakte und auswertungssichere Ausgestaltung der Vorrichtung.

Im Hinblick auf eine besonders einfache Verarbeitung der durch die Vorrichtung erzeugten Meßdaten könnte die Auswerteelektronik eine Einrichtung zur Erzeugung eines elektronischen Signals aufweisen. Hierbei könnte die Auswerteelektronik derart ausgebildet sein, daß das elektronische Signal genau dann erzeugt wird, wenn ein echter, von Störimpulsen diskriminierter Meßimpuls festgestellt wurde. Im einfachsten Fall könnte das Signal ein elektronischer, vorzugsweise digitaler Standardimpuls sein, wobei hier insbesondere an einen TTL-lmpuls gedacht ist.

Ein von der Auswerteelektronik erzeugtes elektronisches Signal könnte zu einer Zentraleinheit übertragbar sein, die vorzugsweise ein z. B. tragbarer Personal Computer ist. In einer derartigen Zentraleinheit könnten die Meßdaten in Form von beispielsweise Laufzeiten der Stoßwellen von der Impulseintragsstelle zum jeweiligen Sensor verarbeitet werden.

Insbesondere im Hinblick auf eine sichere Übertragung des Signals von den Sensoren bzw. der Auswerteelektronik zu der Zentraleinheit könnten die Sensoren und/oder Auswerteelektroniken untereinander elektrisch verbunden sein. Zur elektrischen Verbindung könnten Standardkabel einfachster Art verwendet werden, da hier eine Unterdrückung von Störungen oder Signalverfälschungen nicht erforderlich ist. Es geht hier nur um die sichere Übertragung eines elektronischen Standardsignals, wobei in der Zentraleinheit keine aufwendige Diskriminierung mehr erforderlich ist, da die entscheidende Diskriminierung echter Impulse von Störimpulsen bereits in der Auswerteelektronik stattfindet. Als elektrische Verbindung könnte eine Ringleitung oder sternförmige Leitung vorgesehen sein. Hierbei ist auf den jeweiligen Anwendungsfall abzustellen, wobei bei großen Prüflingen eine Ringleitung aufgrund der insgesamt kürzeren Gesamtkabellänge vorteilhaft sein könnte.

Alternativ zu einer Übertragung über elektrische Leitungen könnte die Übertragung mittels Funkwellen, Ultraschallwellen oder Infrarotstrahlung erfolgen. Hierzu könnte jedem Sensor eine Sender-/Empfängereinheit für Funkwellen, Ultraschallwellen oder Infrarotstrahlung zugeordnet sein. Über eine derartige Sender-/Empfängereinheit könnte insbesondere das elektronische Signal der Auswerteelektronik übertragen werden. Hierbei lassen sich umfangreiche Verkabelungen vermeiden.

In einer konkreten Ausgestaltung könnte jedem Sensor ein Schwingungsdetektor zugeordnet sein. Der Schwingungsdetektor dient zur Detektion mechanischer Impulse. In besonders einfacher Weise könnte der Schwingungsdetektor ein Piezoelement sein.

Zur sicheren Übertragung eines mechanischen und/oder elektrischen Impulses vom Prüfling auf den Sensor könnte jedem Sensor mindestens ein Übertragungsstift für den Impuls zugeordnet sein. Der Übertragungsstift könnte in konstruktiv besonders einfacher Weise ein Metallstift, vorzugsweise Stahlstift, sein. Ein derartiger Übertragungsstift könnte zu Beginn einer Messung in den Prüfling des zu untersuchenden Materials eingebracht werden, worauf der Sensor mit dem Übertragungsstift gekoppelt wird. Hierbei könnte der Sensor in besonders einfacher Weise an dem Übertragungsstift aufgehängt oder eingehängt werden. Besteht das zu untersuchende Material aus beispielsweise Beton, könnte auf einen Übertragungsstift verzichtet werden.

Bei der erfindungsgemäßen Vorrichtung zur Materialuntersuchung wird beispielsweise die Laufzeit eines Impulses von einer Impulseintragsstelle zu jedem einzelnen Sensor gemessen. Dabei ist eine Initialisierung der Sensoren erforderlich. Hierzu könnte jedem Sensor eine Uhr zugeordnet sein. Beispielsweise könnte eine Initialisierung derart erfolgen, daß der erste Sensor, der einen echten Impuls feststellt, seine Uhr startet und dabei gleichzeitig ein vorzugsweise elektronisches Kommunikations-Signal an die anderen Sensoren überträgt, um deren Uhren auf Null zu setzen und ebenfalls zu starten. Dieses Initialisierungsprinzip ist insbesondere dann besonders günstig, wenn der Impulseintrag bzw. die Impuls-einleitung in unmittelbarer Nähe des ersten Sensors erfolgt.

Nach erfolgter Detektion eines echten Impulses sendet die Auswerteelektronik beispielsweise ein elektronisches Signal zur einer Zentraleinheit. Hierbei ist es günstig, wenn jedem Sensor ein individuelles Identifizierungsmittel zugeordnet ist, so daß eine Zuordnung der bei der Zentraleinheit ankommenden Signale zu dem jeweils sendenden Sensor ermöglicht ist. Hierzu könnten sich die Sensoren mit einem eindeutigen Code identifizieren.

In weiter vorteilhafter Weise könnte jedem Sensor ein Speicher für Meßergebnisse zugeordnet sein. Dies ermöglicht ein direktes Auslesen der Meßergebnisse an jedem Sensor.

Des weiteren könnte jedem Sensor eine Anzeigeeinrichtung für Meßergebnisse zugeordnet sein. Hierdurch könnten Meßergebnisse direkt am Sensor abgelesen werden.

Bei einer besonders vorteilhaften Ausgestaltung der Vorrichtung zur Materialuntersuchung könnten mindestens drei Sensoren vorgesehen sein. Hierdurch könnten letztlich dreidimensionale Ergebnisgrafiken hinsichtlich des inneren Zustands des Prüflings erzeugt werden. Je mehr Sensoren hierbei eingesetzt werden, um so detaillierter ist eine derartige Grafik. Dabei ist wesentlich, daß die Sensoren dem Material geometrisch unabhängig voneinander zuordenbar sind. Eine starre Anordnung der Sensoren in beispielsweise Ringform ist nicht erforderlich. Zur wirksamen Auswertung der dreidimensionalen Meßdaten ist es lediglich erforderlich, daß die geometrischen Positionen der Sensoren festgestellt sind.

Im Hinblick auf eine besonders elegante und praktische Impulseinleitung könnte der Sensor oder könnten mehrere Sensoren als Impulsgeber ausgebildet sein. Hierzu könnte mindestens einem Sensor einer Einrichtung zur Impulseinleitung zugeordnet sein. Eine derartige Einrichtung könnte beispielsweise durch ein Piezoelement gebildet sein, das gleichzeitig als Schwingungsdetektor dient. Alternativ oder zusätzlich hierzu könnte die Einrichtung zur Impulseinleitung ein Stift, vorzugsweise Metallstift, sein. Ein derartiger Stift könnte mit dem Sensor über ein Kopplungsstück aus Gummi verbunden sein. Der Stift könnte mittels eines als Hammer ausgebildeten Impulsgebers zur Impulseinleitung aktiviert werden.

Im Hinblick auf eine sichere Messung der durch den Impulsgeber eingeleiteten Impulse ist es wesentlich, daß zwischen eingeleiteten Impulsen und Störimpulsen unterschieden bzw. diskriminiert wird. Störimpulse können beispielsweise bei der Untersuchung eines Baums durch auf einer benachbarten Straße vorbeifahrende Kraftfahrzeuge erzeugt werden. Selbst an dem zu untersuchenden Prüfling vorbeilaufende Personen können Störimpulse über den Boden erzeugen bzw. in den Prüfling einleiten. Daher ist es besonders vorteilhaft, wenn die Sensoren bzw. die Auswerteelektroniken bei jeder Anwendung individuell derartige Störimpulse schon vor der eigentlichen Messung erkennen können. Hierzu könnte die Auswerteelektronik Mittel zur Selbstkalibrierung aufweisen. Dabei wird die Detektionsschwelle der Auswerteelektronik von der Auswerteelektronik selbst auf ein Niveau eingestellt, das oberhalb des Niveaus sämtlicher bisher festgestellter Störimpulse oder Störvibrationen liegt. Dieser Selbstkalibrierungsvorgang könnte kontinuierlich erfolgen, wobei lediglich in der Zeit der eigentlichen Messung keine Selbstkalibrierung erfolgt. Dadurch kann eine wesentliche Anzahl an Störimpulsen von vornherein unterdrückt werden.

Hinsichtlich einer einfachen Feststellung der Positionen der Sensoren relativ zueinander könnten dem Sensor oder den Sensoren Ausziehmaßstäbe zugeordnet sein. Alternativ hierzu könnte dem Sensor oder den Sensoren ein Seil mit einer Winkelanzeige zugeordnet sein. Das Seil verbindet benachbarte Sensoren miteinander. Hierdurch können die Entfernung und der Winkel zum benachbarten Sensor erfaßt werden. Bei bekannter Sensorgröße kann aus den Sensorabständen und Seilverbindungswinkeln die Geometrie des Probenquerschnitts im untersuchten Bereich, beispielsweise ein Baumquerschnitt, angenähert werden.

Alternativ oder zusätzlich hierzu könnte ein Infrarot- oder Laserabstandsmesser vorgesehen sein. In Verbindung mit der Zentraleinheit könnte hierdurch die Position der Sensoren und der Impulseintragsstellen erfaßt und als dreidimensionales Bild dargestellt werden. In diesem Fall kann aus den ermittelten Impulsdaten direkt ein dreidimensionales Bild des inneren Zustands errechnet, dargestellt und ausgegeben werden.

Zum besseren Verständnis der erfindungsgemäßen Lehre sind im folgenden wesentliche Aspekte der erfindungsgemäßen Lehre nochmals erläutert:

Zur Erzeugung dreidimensionaler Zustandsgrafiken werden im Rahmen der erfindungsgemäßen Vorrichtung mindestens drei, vorzugsweise baugleiche, Sensoren um den zu untersuchenden Querschnitt oder Prüfling herum nach wunschgemäßer Geometrie angeordnet. Bei stehenden Bäumen sind beispielsweise meist vier bis sechs Sensoren pro Querschnitt oder Stammabschnitt ausreichend. Die Impulseinleitung kann mittels eines handelsüblichen Hammers erfolgen. Vom jeweiligen Ort der an verschiedenen Stellen erfolgenden Impulseinleitung ergibt sich zu jedem Sensor ein entsprechender Meßwert, woraus sich nach entsprechender Meßwertverarbeitung ein dreidimensionales, quasi-tomografisches Querschnittsbild ergibt. Die Impulseinleitung kann an beliebigen Stellen des Prüflings erfolgen.

Bei einer konkreten Ausführung enthält jeder Sensor eine eigene, selbständige elektronische Regelung bzw. Auswerteelektronik, die gegebenenfalls einen elektronischen Zeitgeber bzw. eine Uhr aufweist. Die aus dem Prüfling des zu untersuchenden Materials ankommenden mechanischen oder elektrischen Impulse werden nach ihrer Umwandlung in Stromimpulse - beispielsweise über Piezokristalle - direkt im Sensor elektronisch verarbeitet und diskriminiert. Äußerliche Störeinflüsse werden also direkt im Sensor unterdrückt. Die Störungen, die bislang im Übertragungssystem - Kabel - problematisch waren, werden hiermit ausgeschlossen. Wird ein aus dem Prüfling ankommender Impuls als korrekt erkannt, wird ein elektronischer, vorzugsweise digitaler Standardimpuls - beispielsweise TTL-lmpuls - erzeugt. Dieser kann über einfache, kostengünstige Standardkabel nahezu unbeschränkter Länge oder auch per Funk, Infrarot oder Ultraschall an andere Sensoren oder an eine Zentraleinheit übertragen werden. Dabei identifizieren sich die Sensoren mit einem eindeutigen Code.

Optional können die Sensoren nicht nur ankommende Impulse feststellen, sondern - beispielsweise über den umgekehrten Piezoeffekt ― auch selbst Impulse erzeugen und einleiten, zumal hierfür prinzipiell die gleiche Technik benötigt wird.

Die Sensoren können beispielsweise in Form einer Ringleitung miteinander verbunden sein. Die Daten können jedoch auch sternförmig oder per Funk zu einer Zentraleinheit mit gegebenenfalls Anzeige, Speicher und Ausgabe oder gleich zu einem vorzugsweise feldgängigen Computer übertragen werden. Dabei ist die Anzahl der Sensoren quasi beliebig, wobei sich diese in vorteilhafter Weise bei der Kommunikation jeweils selbst identifizieren. Die Sensoren müssen lediglich eindeutig identifiziert und ihre Lage zugeordnet werden, wobei bei jedem Impulseintrag die Position des Impulseintrags festgehalten wird.

Bei Bäumen kann es notwendig werden, die Sensoren auf mehrere Meter Höhe anzubringen. Dies kann mittels Teleskopstangen erfolgen, da die Sensoren entweder direkt eingeschlagen oder auf eingeschlagenen Stiften angebracht werden können.

Ein typischer Meßablauf bzw. typisches Meßverfahren könnte wie folgt durchgeführt werden. Zunächst wird die richtige Position der Sensoren am Prüfling bestimmt. Anschließend werden die Sensoren beispielsweise zur Baum- und Holzuntersuchung vorzugsweise auf in das Holz eingeschlagene oder eingeschraubte Stifte aufgesteckt, aufgeschraubt oder aufgeflanscht, damit eine ausreichend stabile Kopplung mit dem Holz gewährleistet ist. Außerdem wird die gegebenenfalls dreidimensionale Geometrie des Prüflings erfaßt, was auf laser- und PC-gestützte Weise erfolgen kann. Mit der Geometrieerfassung wird die Position der Sensoren erfaßt. Zu Beginn einer Messung befinden sich sämtliche Sensoren in einer sogenannten "Wartestellung".

Die Impulseinleitung bzw. der Impulseintrag kann je nach Aufgabenstellung an einer Stelle oder mehreren beliebigen Stellen des Prüflings erfolgen. Zwischen Impulseintragsstelle und jedem Sensor ergibt sich somit eine Meßstrecke mit mindestens einem individuellen Meßwert (z. B. Laufzeit, Leitfähigkeit, Dämpfung). Zu jedem Impulseintrag entsteht folglich eine Liste der Meßwerte für jeden Sensoren. Diese werden der jeweiligen Strecke zwischen Impulseintragsstelle und Sensor zugeordnet.

Bei einer einfachen Ausführungsform hängt über jedem Sensor ein beispielsweise mit Gummi befestigter Schlagstift, über den der Stoßwelleneintrag erfolgen kann, wozu vorzugsweise ein handelsüblicher Hammer verwendet wird.

Im zweiten Arbeitsgang nach Positionierung der Sensoren erfolgt jeweils mindestens ein Schlag auf die angebrachten Impulseintragsstifte. Der Vorteil bei dieser Vorgehensweise besteht darin, daß die Koordinaten des Impulseintrags jeweils durch die ohnehin erfaßten Koordinaten der Sensoren gegeben sind.

Der Impulseintrag kann aber auch - beispielsweise mittels Hammer und/oder Schlagstift - an beliebigen anderen Stellen erfolgen. Zur direkten Bewertung bezüglich innerer Holzschäden reicht dieses Vorgehen bereits aus, da die Ergebnisse vom untersuchenden Fachmann direkt erfaßt und zugeordnet werden können. Soll jedoch ein möglichst vollständiges tomografisches Bild ermittelt und dargestellt werden, muß auch die jeweilige Position des Impulseintrags entsprechend genau erfaßt werden.

Die Sensoren werden entweder durch den Impulseintrag - über eine Kabelverbindung zwischen Hammer und Sensoren - oder durch den ersten Sensor initialisiert, der einen ankommenden Impuls identifiziert hat. Initialisierte Sensoren starten ihre internen Uhren und bestimmen z. B. die Zeitdifferenz bis der nächste Impuls bei ihnen ankommt. Die Übertragung der elektronischen Standardimpulse über Kabel oder Funk erfolgt fast per Lichtgeschwindigkeit und ist damit um Größenordnungen schneller als die Übertragung von Stoßwellen. Daher wirkt sich der zeitliche Versatz durch die elektronische Übertragung der Initialisierung nicht signifikant auf die Meßgenauigkeit aus.

Jeder Sensor sendet die Zeit zwischen Initialisierung und Impulsdetektion an die anderen Sensoren und/oder an die Zentraleinheit, wo die Werte gesammelt werden.

Die Ergebnisse der jeweiligen Laufzeiten werden vorzugsweise direkt auf Papier ausgedruckt oder auf einem feldgängigen, beispielsweise wasserdichten Display angezeigt.

Die Auswertung kann per Computer unterstützt werden, indem das Display mit einem solchen verbunden oder direkt ein feldgängiger Personalcomputer verwendet wird. Stoßwelleneintragspunkt und Sensorpositionen werden entweder manuell oder grafisch eingetragen oder sie ergeben sich automatisch daraus, daß der Sensor, neben dem geschlagen wurde, die Laufzeit Null meldet.

Aus den Laufzeiten für die jeweiligen Verbindungsstrecken zwischen Impulseintrag und Sensor ergeben sich automatisch quasi-tomografische Querschnittsbilder des Zustands im Prüfling. Die Anzahl der Verbindungsstrecken und damit der Ergebnisse ergibt sich in Abhängigkeit von der Anzahl n der Sensoren wie folgt:

Erfolgt der Impulseintrag nicht direkt an einem der Sensoren, so entstehen pro Impuls n Laufzeitergebnisse, wohingegen n-1 Ergebnisse entstehen, wenn der Impulseintrag direkt an einem Sensor erfolgt.

Wird an jedem Sensor ein Impulseintrag vorgenommen, so entstehen n(n-1) Ergebnisse. Bei nur sechs Sensoren und damit sechs Schlageintragspunkten an einem Baum entstehen also dreißig Meßstrecken mit entsprechender Laufzeitinformation, woraus sich ein quasi-tomografisches Querschnittsbild ergibt. Da pro Impulseintrag nur wenige Sekunden benötigt werden, kann auf diesem Weg in sehr kurzer Zeit eine umfassende Information über den inneren Zustand ermittelt werden.

Wenn beispielsweise zwei Sensorringe in unterschiedlichen Höhen an beispielsweise einem stehenden Baum angebracht werden - beispielsweise auf Fuß- und in Kopfhöhe -, ergeben sich automatisch umfangreiche Daten über den Zustand des gesamten Volumens zwischen diesen beiden Ringen. Aus zwei Ringen mit jeweils sechs Sensoren entstehen bei zwölf Impulseinträgen - je einer neben den Sensoren - insgesamt 132 Verbindungsstrecken mit entsprechender Laufzeitinformation, woraus sich ein relativ genaues Bild über den Holzzustand zusammensetzen läßt.

Die erfindungsgemäße Vorrichtung ist dabei nicht an eine bestimmte Anordnung der Sensoren - beispielsweise um einen Querschnitt herum - gebunden. Vielmehr können die Sensoren frei positioniert werden. Da auch die Anzahl der Sensoren nicht beschränkt ist, kann damit auch die Genauigkeit der dreidimensionalen Erfassung des Prüflings bestimmt werden, da die Genauigkeit durch Anzahl und Position der Sensoren sowie Anzahl der Impulseinträge bestimmt ist. Eine EDV-gestützte Erfassung der Position von Sensoren und Impulseintrag sowie eine entsprechend automatisierte Auswertung ermöglicht eine leichte Verarbeitung der mit der Anzahl der Sensoren schnell ansteigenden Anzahl der Ergebniswerte, wobei einzig die Geometrie der Probe und die Anordnung der Sensoren erfaßt bzw. eingegeben werden muß.

Die Geometrie bzw. Oberflächen-Topologie des Prüflings und die Lage der Sensoren sind Grundlage weiterer Auswertungen. Die Genauigkeit ihrer Erfassung bestimmt die Genauigkeit und Aussagekraft der Ergebnisse. Diese Erfassung kann dabei skizzenhaft erfolgen oder über handelsübliche Entfernungsmessinstrumente. Ebenso sind Laserentfemungs- und Positionserfassungsgeräte sinnvoll.

Mit der erfindungsgemäßen Vorrichtung kann die zeitliche und räumliche Intensitätsverteilung der Impulse gemessen werden. Dabei können nicht nur Einzelimpulse eingetragen und deren Ankunft erfaßt werden, sondern auch Impulsserien mit gleicher oder schwankender Intensität und Frequenz.

Die Auswerteelektronik umfaßt Mittel zur Diskriminierung des Impulses von Störimpulsen, wobei hier eine entsprechende Software integriert sein könnte.

In besonders vorteilhafter Weise ist die Vorrichtung für einen Einsatz im Freien wasserdicht ausgebildet. Hierdurch ist ein langer und störungsfreier Betrieb der Vorrichtung gewährleistet.

Die Sensoren könnten nicht nur auf einen Stift, sondern auf eine sternförmige Stiftkombination aufgesetzt werden, die in verschiedene Richtungen zeigende Stiftspitzen aufweist. Wenn nun die ankommenden Impulse je nach Einzelspitze getrennt erfaßt werden, können Ergebnisse über die räumliche Richtung der Impulse ermittelt werden, was beispielsweise bei Bäumen sehr interessant ist, da es hier unterschiedliche radiale und tangentiale Ausbreitungsverhalten gibt.

Der Schlagstift, der entweder von einem Hammer oder von einem Sensor betätigt werden kann, kann in unterschiedliche Richtung orientiert sein. Hierdurch kann einem unterschiedlichen Ausbreitungsverhalten im Material Rechnung getragen werden.

Bei der erfindungsgemäßen Vorrichtung ermöglichen mehrere Sensoren quasi-tomografische Bestimmungen des inneren Zustands mit lediglich wenigen Messungen. Dabei wird nur noch eine Sorte von gleich eingestellten Sensoren benötigt, womit die Herstellungskosten reduziert werden. Die Sensoren werten Impulse aus dem zu untersuchenden Material direkt, eigenständig und damit "vor Ort" aus, was Störquellen und Aufwand vermindert. Der Schlageintrag kann beispielsweise mittels eines handelsüblichen Hammers erfolgen, was sehr flexibel und kostengünstig ist. Die Sensorverbindung benötigt nur noch einfache, handelsübliche und kostengünstige Verbindungskabel oder kann per Funk oder anderen Femübertragungsmethoden erfolgen, weil nur noch elektronische Standardimpulse übertragen werden, die hinsichtlich externer Störungen unempfindlich sind.

Aufgrund der technischen Eigenschaften des Systems können beliebig viele Sensoren am Prüfling uneingeschränkt positioniert werden. Die Vorrichtung ist aufgrund der Sensorabschirmung, Standardkabel und der übertragenen Impulse äußerst unempfindlich gegen elektromagnetische Störstrahlungen, falsche Handhabung, mechanische Belastungen und andere Störungen.

Quasi-tomografische Querschnittsbilder entstehen automatisiert und auf sehr einfachem Weg.

Volumenrelevante Ergebnisse entstehen schon bei der Verwendung mit drei Sensoren. Eine fast beliebige dreidimensionale Erfassung des Zustands ist auf einfachem Weg mit mehreren Sensoren möglich. Eine Messung kann sehr schnell erfolgen, indem zunächst die Stifte eingesteckt werden, dann die Sensoren montiert werden und anschließend ein Schlagen oder anderes Impulsgeben erfolgt. Schließlich werden die Meßergebnisse notiert bzw. aufgenommen. Für die hierzu notwendigen zwei Baumumrundungen werden nur wenige Minuten benötigt.

Es gibt nun verschiedene Möglichkeiten, die Lehre der vorliegenden Erfindung in vorteilhafter Weise auszugestalten und weiterzubilden. Dazu ist einerseits auf die nachgeordneten Patentansprüche, andererseits auf die nachfolgende Erläuterung eines Ausführungsbeispiels der erfindungsgemäßen Vorrichtung anhand der Zeichnung zu verweisen. In Verbindung mit der Erläuterung des bevorzugten Ausführungsbeispiels der erfindungsgemäßen Vorrichtung anhand der Zeichnungen werden auch im allgemeinen bevorzugte Ausgestaltungen und Weiterbildungen der Lehre erläutert. In der Zeichnung zeigen
- Fig. 1: in einer schematischen Darstellung das Ausführungsbeispiel der erfindungsgemäßen Vorrichtung zur Materialuntersuchung im an einem Baum angeordneten Zustand und
- Fig. 2: in einer schematischen Draufsicht einen Sensor des Ausführungsbeispiels aus Fig. 1.

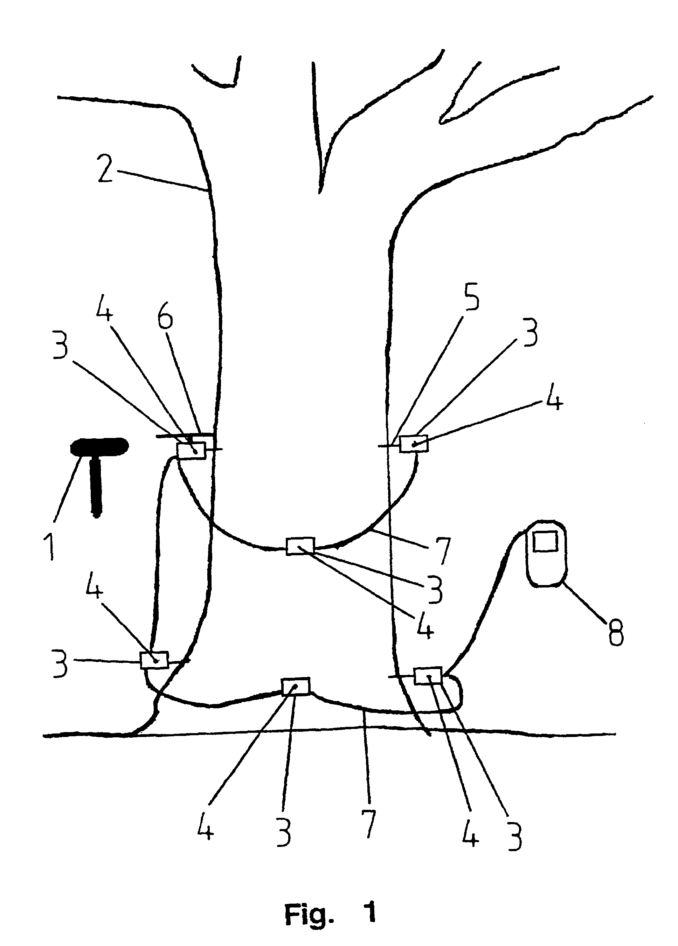

Fig. 1 zeigt in einer schematischen Darstellung das Ausführungsbeispiel der erfindungsgemäßen Vorrichtung zur Materialuntersuchung. Die Vorrichtung weist einen als Hammer ausgebildeten Impulsgeber 1 zur Erzeugung eines in das Material 2 einleitbaren Impulses auf. Als zu untersuchendes Material 2 dient ein Baum. Die Vorrichtung weist des weiteren sechs dem Material 2 zugeordnete Sensoren 3 zur Detektion des Impulses mit sechs Auswerteelektroniken 4 zur Diskriminierung des Impulses von Störimpulsen auf. Im Hinblick auf eine universelle Anwendung der Vorrichtung auch bei großen Prüflingen ist jedem Sensor 3 eine separate Auswerteelektronik 4 zugeordnet.

Die Auswerteelektronik 4 führt die Diskriminierung echter, eingeleiteter Impulse von Störimpulsen durch. Hierbei sind keine langen, Kommunikationsstrecken zwischen dem Sensor 3 bzw. einem Schwingungsdetektor und der Auswerteelektronik 4 erforderlich. Die Auswerteelektronik 4 ist bei dem gezeigten Ausführungsbeispiel in den Sensor 3 integriert. Die Sensoren 3 sind mittels eines Übertragungsstifts 5 mit dem Material 2 gekoppelt. Dabei sind die Sensoren 3 am Übertragungsstift 5 auf- oder eingehängt. Einem der Sensoren 3 ist beispielhaft des weiteren eine Einrichtung zur Impulseinleitung in Form eines Stifts 6 zugeordnet. Die Impulseinleitung erfolgt durch einen Schlag mit dem Hammer auf den Stift 6.

Die Sensoren 3 sind über Verbindungskabel 7 miteinander verbunden. Des weiteren ist eine Verbindung zwischen den Sensoren 3 und einer Zentraleinheit 8 vorgesehen. Zur Zentraleinheit 8 werden elektronische Signale übermittelt, die von den Auswerteelektroniken 4 der jeweiligen Sensoren 3 erzeugt werden, wenn ein echter Impuls detektiert worden ist. Dabei wird ein für jeden Sensor 3 individueller Code übermittelt, so daß der Ankunftsort des detektierten Impulses durch die Zentraleinheit 8 zugeordnet werden kann.

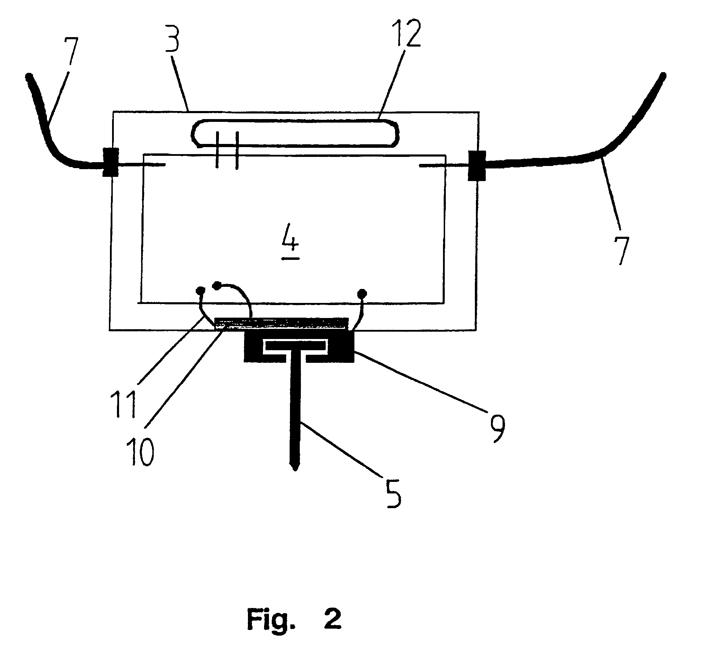

Fig. 2 zeigt in einer schematischen Draufsicht einen Sensor 3 des Ausführungsbeispiels aus Fig. 1. Der Sensor 3 weist eine integrierte Auswerteelektronik 4 auf. Der zur Kopplung mit dem Material dienende Stift 5 ist über ein Kopplungsstück 9 an den Sensor 3 angekoppelt. Direkt in Verbindung mit dem Kopplungsstück 9 ist ein Schwingungsdetektor 10 vorgesehen, der durch ein Piezoelement gebildet wird. Die Auswerteelektronik 4 und der Schwingungsdetektor 10 sind über eine elektrische Verbindung 11 gekoppelt.

Sowohl der Übertragungsstift 5 als auch das Kopplungsstück 9 können aus einem leitfähigen Material, vorzugsweise Metall, hergestellt sein. Dies gewährleistet nicht nur eine Schwingungsübertragung vom Material 2 zum Schwingungsdetektor 10 sondern auch eine Übertragung elektrischer Impulse zum Schwingungsdetektor 10 und über die elektrische Verbindung 11 zur Auswerteelektronik 4. Somit können nicht nur mechanische sondern auch elektrische Impulse mit dem Sensor 3 detektiert werden.

Die Übertragung eines elektronischen Signals, das durch eine spezielle, der Auswerteelektronik 4 zugeordnete Einrichtung erzeugt wird, oder sonstiger Signale vom Sensor 3 kann alternativ zur Kabelübertragung mittels Kabeln 7 über eine Sender/Empfängereinheit 12 für Funkwellen, Ultraschallwellen oder Infrarotstrahlung erfolgen. Mittels der Sender-/Empfängereinheit 12 ist auch die Initialisierung der Sensoren 3 zu Beginn einer Messung möglich. Auf Verbindungskabel 7 kann in diesem Fall verzichtet werden. Die Sender-/Empfängereinheit 12 kann zur Übertragung jedweder Signale, Messergebnisse oder dergleichen dienen.

Die Auswerteelektronik 4 dient als selbständige Einheit mit einer zentralen Prozeßeinheit.

Hinsichtlich weiterer vorteilhafter Ausgestaltungen der erfindungsgemäßen Vorrichtung zur Materialuntersuchung wird zur Vermeidung von Wiederholungen auf den allgemeinen Teil der Beschreibung sowie auf die beigefügten Patentansprüche verwiesen.

Schließlich sei ausdrücklich darauf hingewiesen, daß das voranstehend beschriebene Ausführungsbeispiel der erfindungsgemäßen Vorrichtung lediglich zur Erörterung der beanspruchten Lehre dient, diese jedoch nicht auf dieses Ausführungsbeispiel einschränkt.

## Patentansprüche

1. Vorrichtung zur Materialuntersuchung, insbesondere von Bäumen, sonstigen Hölzern und Beton, mit einem Impulsgeber (1) zur Erzeugung eines in das Material (2) einleitbaren Impulses, mindestens einem dem Material (2) zuordenbaren Sensor (3) zur Detektion des Impulses und einer Auswerteelektronik (4) zur Diskriminierung des Impulses von Störimpulsen, wobei jedem Sensor (3) eine Auswerteelektronik (4) zugeordnet ist,
**dadurch gekennzeichnet, daß** die Auswerteelektronik (4) unmittelbar neben dem Sensor (3) angeordnet oder in den Sensor (3) integriert ist.

2. Vorrichtung nach Anspruch 1, **dadurch gekennzeichnet, daß** der Impuls ein mechanischer und/oder elektrischer Impuls ist.

3. Vorrichtung nach Anspruch 1 oder 2, **dadurch gekennzeichnet, daß** die Auswerteelektronik (4) eine Einrichtung zur Erzeugung eines elektronischen Signals aufweist, wobei vorzugsweise das Signal ein elektronischer, vorzugsweise digitaler Standardimpuls ist.

4. Vorrichtung nach Anspruch 3, **dadurch gekennzeichnet, daß** das Signal zu einer Zentraleinheit (8), vorzugsweise ein Personal-Computer, übertragbar ist.

5. Vorrichtung nach einem der Ansprüche 1 bis 4, **dadurch gekennzeichnet, daß** die Sensoren (3) untereinander elektrisch verbunden sind, wobei vorzugsweise die Verbindung durch eine Ringleitung oder sternförmige Leitung realisiert ist.

6. Vorrichtung nach Anspruch 4 oder 5, **dadurch gekennzeichnet, daß** die Übertragung mittels einer Kabelverbindung, Funkwellen, Ultraschallwellen oder Infrarotstrahlung durchführbar ist.

7. Vorrichtung nach einem der Ansprüche 1 bis 6, **dadurch gekennzeichnet, daß** jedem Sensor eine Sender-/Empfängereinheit (12) für Funkwellen, Ultraschallwellen oder Infrarotstrahlung zugeordnet ist und/oder daß jedem Sensor (3) ein Schwingungsdetektor (10), vorzugsweise ein Piezoelement, zugeordnet ist.

8. Vorrichtung nach einem der Ansprüche 1 bis 7, **dadurch gekennzeichnet, daß** jedem Sensor (3) mindestens ein Übertragungsstift (5), vorzugsweise ein Metallstift, vorzugsweise Stahlstift, für den Impuls zugeordnet ist und/oder daß jedem Sensor (3) eine Uhr und/oder ein identifizierungsmittel zugeordnet ist.

9. Vorrichtung nach einem der Ansprüche 1 bis 8, **dadurch gekennzeichnet, daß** jedem Sensor (3) ein Speicher für Meßergebnisse zugeordnet ist und/oder daß jedem Sensor (3) eine Anzeigeeinrichtung für Meßergebnisse zugeordnet ist.

10. Vorrichtung nach einem der Ansprüche 1 bis 9, **dadurch gekennzeichnet, daß** mindestens drei Sensoren (3) vorgesehen sind und/oder daß die Sensoren (3) dem Material (2) geometrisch unabhängig voneinander zuordenbar sind.

11. Vorrichtung nach einem der Ansprüche 1 bis 10, **dadurch gekennzeichnet, daß** der Sensor (3) oder mehrere Sensoren (3) als Impulsgeber (1) ausgebildet ist oder sind und/oder daß mindestens einem Sensor (3) eine Einrichtung zur Impulseinleitung, vorzugsweise ein Stift (6), vorzugsweise Metallstift, zugeordnet ist.

12. Vorrichtung nach einem der Ansprüche 1 bis 11, **dadurch gekennzeichnet, daß** der Impulsgeber (1) ein Hammer ist und/oder daß die Auswerteelektronik (4) Mittel zur Selbstkalibrierung aufweist.

13. Vorrichtung nach einem der Ansprüche 1 bis 12, **dadurch gekennzeichnet, daß** dem Sensor (3) oder den Sensoren (3) Ausziehmaßstäbe und/oder ein Seil mit einer Winkelanzeige zugeordnet sind oder ist und/oder daß ein Infrarot- oder Laserabstandsmesser vorgesehen ist.

## Claims

1. Apparatus for investigating materials, especially trees, other woods and concrete, having a pulse generator (1) for generating a pulse that can be introduced into the material (2), at least one sensor (3), which can be associated with the material (2), for detecting the pulse, and an electronic evaluator (4) for discriminating between the pulse and interference pulses, an electronic evaluator (4) being associated with each sensor (3), **characterised in that** the electronic evaluator (4) is arranged immediately next to the sensor (3) or is integrated in the sensor (3).

2. Apparatus according to claim 1, **characterised in that** the pulse is a mechanical and/or an electrical pulse.

3. Apparatus according to claim 1 or 2, **characterised in that** the electronic evaluator (4) has a device for generating an electronic signal, the signal preferably being an electronic, preferably digital, standard pulse.

4. Apparatus according to claim 3, **characterised in that** the signal can be transmitted to a central processing unit (8), preferably a personal computer.

5. Apparatus according to any one of claims 1 to 4, **characterised in that** the sensors (3) are connected to one another electrically, the connection preferably being brought about by a ring circuit or a star-shaped circuit.

6. Apparatus according to claim 4 or 5, **characterised in that** the transmission can be carried out by means of a cable connection, radio waves, ultrasound waves or infrared radiation.

7. Apparatus according to any one of claims 1 to 6, **characterised in that** a transmitter/receiver unit (12) for radio waves, ultrasound waves or infrared radiation is associated with each sensor and/or **in that** a vibration detector (10), preferably a piezo element, is associated with each sensor (3).

8. Apparatus according to any one of claims 1 to 7, **characterised in that** at least one transmission pin (5), preferably a metal pin, preferably a steel pin, for the pulse is associated with each sensor (3) and/or **in that** a clock and/or an identification means is associated with each sensor (3).

9. Apparatus according to any one of claims 1 to 8, **characterised in that** a memory for measurement results is associated with each sensor (3) and/or **in that** a display device for measurement results is associated with each sensor (3).

10. Apparatus according to any one of claims 1 to 9, **characterised in that** at least three sensors (3) are provided and/or **in that** the sensors (3) can be associated with the material (2) geometrically independently of one another.

11. Apparatus according to any one of claims 1 to 10, **characterised in that** the sensor (3) or several sensors (3) is or are in the form of (a) pulse generator(s) (1) and/or **in that** a device for introducing pulses, preferably a pin (6), preferably a metal pin, is associated with at least one sensor (3).

12. Apparatus according to any one of claims 1 to 11, **characterised in that** the pulse generator (1) is a hammer and/or **in that** the electronic evaluator (4) has means for self-calibration.

13. Apparatus according to any one of claims 1 to 12, **characterised in that** pull-out measuring rods and/or a cord having an angle indicator are or is associated with the sensor (3) or the sensors (3) and/or **in that** an infrared or laser distance meter is provided.

## Revendications

1. Dispositif pour essai de matériaux, en particulier d'arbres, de bois quelconques et de béton, avec un émetteur d'impulsions (1) pour engendrer une impulsion pouvant être introduite dans le matériau (2), au moins un détecteur (3) pouvant être adjoint au matériau (2) pour la détection de l'impulsion et une électronique d'exploitation (4) pour la discrimination de l'impulsion d'avec des impulsions parasites, une électronique d'exploitation (4) étant adjointe à chaque capteur (3),
**caractérisé en ce que** l'électronique d'exploitation (4) est disposée immédiatement à côté du capteur (3) ou est intégrée dans le capteur (3).

2. Dispositif selon la revendication 1,
**caractérisé en ce que** l'impulsion est une impulsion mécanique et/ou électrique.

3. Dispositif selon la revendication 1 ou 2,
**caractérisé en ce que** l'électronique d'évaluation (4) présente un dispositif pour engendrer un signal électronique, le signal étant de préférence une impulsion standard électronique, de préférence numérique.

4. Dispositif selon la revendication 3,
**caractérisé en ce que** le signal peut être transféré à une unité centrale (8), de préférence un ordinateur personnel.

5. Dispositif selon l'une des revendications 1 à 4,
**caractérisé en ce que** les capteurs (3) sont reliés électriquement les uns aux autres, la liaison étant de préférence réalisée sous forme d'une ligne annulaire ou d'une ligne en forme d'étoile.

6. Dispositif selon la revendication 4 ou 5,
**caractérisé en ce que** la transmission peut être effectuée au moyen d'une liaison par câble, d'ondes radio, d'ondes ultrasoniques ou d'un rayonnement infrarouge.

7. Dispositif selon l'une des revendications 1 à 6,
**caractérisé en ce qu'**une unité émetteur/récepteur (12) pour ondes radio, ondes ultrasoniques ou rayonnement infrarouge est adjointe à chaque capteur et/ou qu'un détecteur de vibrations (10), de préférence un élément piézo, est adjoint à chaque capteur (3).

8. Dispositif selon l'une des revendications 1 à 7,
**caractérisé en ce qu'**au moins une pointe de transmission (5), de préférence une pointe de métal, de préférence une pointe d'acier, sont adjoints pour l'impulsion et/ou qu'une horloge et/ou un moyen d'identification est adjoint à chaque capteur (3).

9. Dispositif selon l'une des revendications 1 à 8,
**caractérisé en ce qu'**une mémoire pour des résultats de mesure est adjointe à chaque capteur (3) et/ou qu'un dispositif d'affichage pour des résultats de mesure est adjoint à chaque capteur (3).

10. Dispositif selon l'une des revendications 1 à 9,
**caractérisé en ce qu'**au moins trois capteurs (3) sont prévus et/ou que les capteurs (3) peuvent être adjoints géométriquement indépendamment les uns des autres au matériau (2).

11. Dispositif selon l'une des revendications 1 à 10,
**caractérisé en ce que** le capteur (3) ou plusieurs capteurs (3) est ou sont réalisés sous forme d'émetteur d'impulsions (1) et/ou qu'un dispositif pour l'introduction d'impulsions, de préférence une pointe (6), de préférence une pointe de métal, est adjoint à au moins un capteur (3).

12. Dispositif selon l'une des revendications 1 à 11,
**caractérisé en ce que** l'émetteur d'impulsions (1) est un marteau et/ou que l'électronique d'exploitation (4) présente un moyen d'auto-calibrage.

13. Dispositif selon l'une des revendications 1 à 12,
**caractérisé en ce que** des échelles télescopiques et/ou un câble avec un indicateur d'angle sont adjoints au capteur (3) ou aux capteurs (3) et/ou qu'un dispositif de mesure d'écartement infrarouge ou laser est prévu.
